Europäisches Patentamt

European Patent Office

Office européen des brevets

(11) Publication number: **0 325 032**
**A1**

## (12) EUROPEAN PATENT APPLICATION

(21) Application number: 88310975.3

(22) Date of filing: 21.11.88

(51) Int. Cl.⁴: **B01D 15/00** , **C12Q 1/68**

(30) Priority: 20.11.87 JP 293267/87

(43) Date of publication of application:
26.07.89 Bulletin 89/30

(84) Designated Contracting States:
CH DE FR GB LI SE

(71) Applicant: MITSUI TOATSU CHEMICALS,
INCORPORATED
2-5 Kasumigaseki 3-chome
Chiyoda-Ku Tokyo 100(JP)

(72) Inventor: Iino, Shinji
4-8-17, Sakomachi Hikoshima
Shimonoseki-shi Yamaguchi-ken(JP)
Inventor: Ishigaki, Kyoichi
6-2-45, Sakomachi Hikoshima
Shimonoseki-shi Yamaguchi-ken(JP)
Inventor: Yamamoto, Kumiko
A-201, Fuji Haitsu 819-1, Nabekurahigashi
Konan-ku Ube-shi Yamaguchi-ken(JP)

(74) Representative: Harvey, David Gareth et al
Graham Watt & Co. Riverhead
Sevenoaks Kent TN13 2BN(GB)

(54) Method for separating nucleic acid.

(57) A solution of a mixture containing a nucleic acid to be separated is passed through a column packed with granular hydroxyapatite to retain the nucleic acid on the column, the nucleic acid then being selectively eluted by a solution of an ammonium salt of carbonic acid, to enable the nucleic acid to be effectively separated at high purity. Since hydroxyapatite is used for the packing material, the eluted nucleic acid fraction does not contain inorganic salts except the eluent component which, being an ammonium salt of carbonic acid can be decomposed into ammonia and carbon dioxide by treating under reduced pressure and/or with warming, and thus can be easily and quickly removed from the fraction.

EP 0 325 032 A1

## METHOD FOR SEPARATING NUCLEIC ACID

The present invention relates to a method for separating nucleic acid and, more particularly, to a method for separating a nucleic acid by liquid chromatography.

Remarkable developments have recently been achieved in the field of biochemistry. Particularly in the field of genetic engineering, it is strongly desired to provide a method for separating and/or purifying the nucleic acid within shortened periods of time, at reduced cost.

Various methods have traditionally been used to separate and/or purify a nucleic acid. Among these methods, density-gradient centrifugation (hereinafter abbreviated as an ultracentrifugal method) and liquid chromatography have been well known. The ultracentrifugal method is used, for example, to separate and/or purify a plasmid DNA and to separate single stranded DNA and double stranded DNA.

The ultracentrifugal method is a means for separating the respective component such as the nucleic acids mentioned above by utilizing a slight difference in density of each component. The method, however, has the following disadvantages.

That is, the method uses an ultracentrifuge at an ultra high speed revolution of 20,000 - 50,000 r.p.m. and requires a long-term treatment for 20 -50 hours, at the minimum for 20 hours, although the time is different from case to case. Furthermore, the ultracentrifuge is very expensive and is not used widely in the field of biochemistry. As a result, the equipment is in low frequency of use and thus inevitably is expensive.

The method also requires to use chemicals such as cesium chloride and ethidium bromide which are expensive and strongly toxic to humans.

As mentioned above, the separation and purification of the nucleic acid by the ultracentrifugal method involves a long period of time and high cost, and also poses problems of health risks to operators.

On the other hand, the separation and purification method by liquid chromatography is also generally well known. Hydroxyapatite, BND-cellulose (Benzoylated-Naphthoylated DEAE-cellulose) or the like is used as a column packing material for the separation and purification of the nucleic acid.

The separation and purification method using liquid chromatography has conventionally and generally been used in the field of biochemistry. The operation can be, generally, completed within an hour when high performance liquid chromatography is, in particular, used. An aqueous solution containing phosphates and sodium chloride can be used for elution. It is unnecessary, in particular, to handle expensive and highly toxic materials.

Therefore, the method of liquid chromatography can separate and purify the nucleic acid economically and safely within a much shorter period of time as compared to the ultracentrifugal method. The chromatographic method is also advantageous in that it is relatively easy to treat a large amount of samples.

However, in the conventional methods using liquid chromatography, the aqueous solution containing inorganic salts such as phosphates and sodium chloride is used as an eluent as mentioned above. Therefore the nucleic acid fraction is obtained as a mixed solution with the inorganic salts after separation by liquid chromatography. In the use of the nucleic acid fraction containing the inorganic salts, contaminated inorganic salts cause various troubles. Thus removal of the inorganic salts is needed, for example by dialysis. However, there is a problem that the dialysis treatment usually requires a relatively long period of time, generally about 1 - 2 days.

Although the separation and purification themselves by liquid chromatography, in particular high performance liquid chromatography, can be completed within a relatively short period of time, in consideration of the total period of time including the subsequently needed dialysis for desalting, the separation treatment using conventional liquid chromatography is a long-term operation.

As stated above, there is a problem that a long period of time is required for the separation and purification of the nucleic acid by either of the two conventional methods discussed above.

An object of this invention is thus to provide a method for separating a nucleic acid, which can retain the advantages of liquid chromatography methodology mentioned above and which can reduce the total operation time for the separation or purification.

In consideration of this above problem, we have carried out an extensive investigation into ways of improving the method for separating and purifying the nucleic acid. In the investigation, hydroxyapatite has been applied to a packing material of a column in liquid chromatography and an aqueous solution of ammonium salt of carbonic acid has been tried as an eluent. As a result, it has been found that the nucleic acid can be economically and safely separated in high purity within a short period of time, thereby the present invention has been completed.

In the nucleic acid separation steps by liquid chromatography in this invention, hydroxyapatite is used as the packing material of the column in combination with the aqueous solution of ammonium salt of carbonic acid as the eluent.

According to the method of this invention, the ammonium salt of carbonic acid is dissolved together with the nucleic acid in the purified fraction of the nucleic acid. The ammonium salt of carbonic acid can be decomposed into ammonia and carbon dioxide under reduced pressure and/or by warming the mixture. Therefore ammonia and carbon dioxide can be removed from the fraction simply by vaporizing, and within a short period of time.

As stated above, known methods employ an aqueous solution of non-volatile salts such as phosphates and sodium chloride, and hence desalting must inevitably be conducted by the complex treatment of dialysis over a long period of time, generally for 20 - 50 hours. On the other hand, the method of this invention can remarkably reduce the period of time required for the recovery and purification of the fractionated nucleic acid.

The ability for the separation and purification of the nucleic acid by the method of this invention is, at least, equivalent to that of the conventional methods using the aqueous phosphate buffer as the eluent. This is clearly shown in Example 1 and Comparative Example 1 which are described below. In the method of this invention, the ammonium salt of carbonic acid contained in the purified fraction of the nucleic acid is subjected to reduced pressure and/or heating, to decompose it into ammonia gas and carbon dioxide, which are removed by vaporizing from the purified fraction. The vaporization operation does not have an adverse effect at all on the quality of nucleic acid which is physically unstable. This effect is clearly supported, as illustrated by Example 2, by the fact that restriction enzyme cleaved the closed-circular plasmid DNA only at one specific site.

As stated above in detail, when the separation or purification of the nucleic acid is conducted by the method of this invention, the separation or purification operation can be completed in a very short period of time as compared to that of conventional methods, namely the ultracentrifugal method and liquid chromatography using a solution of non-volatile salts as the eluent. The purity of the nucleic acid obtained by the method of this invention is also equivalent or superior to that of the nucleic acid purified by the previously known methods. Furthermore, the method of this invention has no tendency adversely to affect on the properties of nucleic acid which is physically unstable. Therefore, the present invention makes a great contribution to the progress in the field of biotechnology including genetic engineering.

The invention will now be described in more detail in the following non-limitative description which is given by way of example with reference to the drawings, in which:

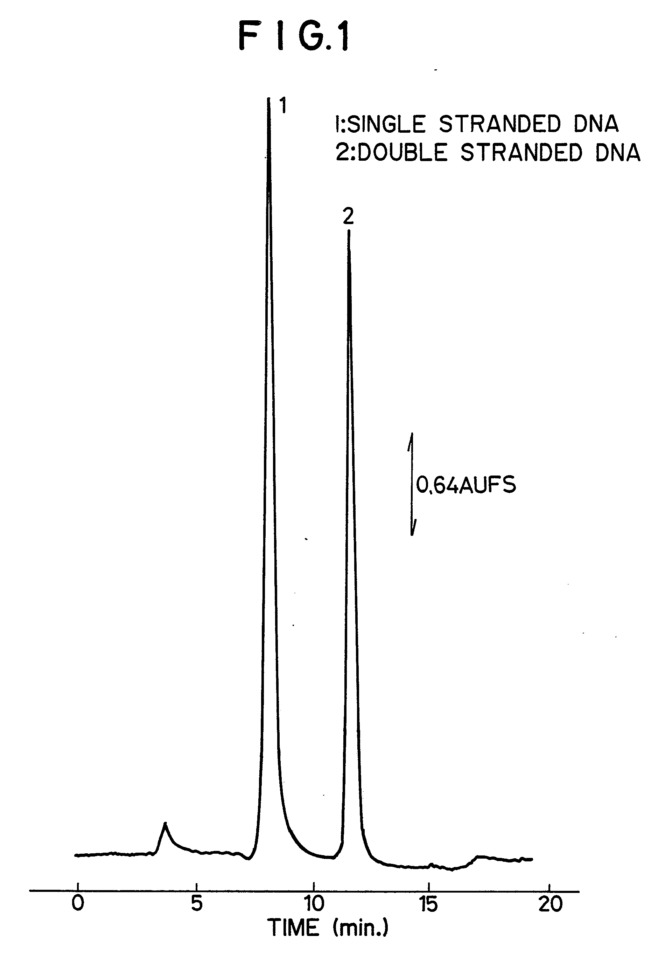

Figures 1 - 3 show peaks of eluted nucleic acids obtained by liquid chromatography in Example 1, Example 2 and Comparative Example 1, respectively.

The method for separating a nucleic acid in this invention comprises the steps of:

(a) passing a solution of a mixture containing the nucleic acid through a column packed with granular hydroxyapatite to retain the nucleic acid in the column;

(b) passing a solution of an ammonium salt of carbonic acid through the column retaining the nucleic acid to selectively elute the nucleic acid retained in the column; and

(c) collecting a fraction of the eluted nucleic acid to recover the nucleic acid from the fraction.

According to the method of this invention, the nucleic acid can be separated and recovered in a state of high purity. Thus the method of this invention can be applied, for example, to the purification of crude nucleic acid containing impurities or to the separation of various nucleic acids in a mixture.

Hydroxyapatite used in the method of this invention is a compound having the formula (I) below. The atomic ratio Ca/P is theoretically 10/6 = 1.67 and practically not always coincides with this value. Generally known compositions have the ratio in the range of about 1.3 - 1.9.

$$Ca_{10}(PO_4)_6(OH)_2 \qquad (I)$$

The site of Ca may be replaced by other metal atoms such as Sr, Ba, Pb, Cd, Na, K, Al and the like. The site of ($PO_4$) may be replaced by acid-radicals such as $SO_4$, $AsO_4$, $VO_4$, $CO_3$ etc. The site of (OH) may be replaced by anion atoms or groups such as F, Cl, $CO_3$ and the like. Therefore there are many sorts of hydroxyapatite having various compositions depending upon the synthetic conditions and history of the compounds.

The hydroxyapatite used in the method of this invention includes all compounds mentioned above and any type of hydroxyapatite which can be used in liquid chromatography may be used in this invention. In typical examples of hydroxyapatite used, the atomic ratio Ca/P mentioned above may be in the range of 1.40 - 1.7 and the average particle size may be in the range of 2 - 20 μm.

About the hydroxyapatite used in this invention, the ($PO_4$) site and/or (OH) site in the above formula (I) may be replaced by $CO_3$. The so-called carbonate apatite thus obtained is more preferable for use in the packing material of chromatography because of better durability.

The method of this invention requires the use of the aqueous solution of an ammonium salt of carbonic acid as the eluent. The aqueous solution is generally used in a concentration ranging from 2 mM to 2 M (in the range of pH 4 - 10).

The ammonium salts of carbonic acid which may be used in this invention includes, for example, ammonium carbonate, ammonium hydrogen carbonate and ammonium carbamate. The particularly preferred salt of carbonic acid is ammonium hydrogen carbonate and ammonium carbamate for the reason described below.

The separation method by the liquid chromatography will be briefly described below.

Liquid chromatography is well known technique. A packing material (adsorbent) is charged into a cylinder as the column. An eluent suitable for the sample to be separated or purified is continuously passed through the packed column and then the sample composed of a mixture of various components is fed to the eluent. Each component in the sample has a different traveling speed in the column, and thus each component is eluted individually. When the technique of the liquid chromatography is employed, each component of the sample can be separated (collected) and purified by utilizing the difference of traveling speed.

By adding alcohol to the purified fraction as an aqueous nucleic acid solution collected through the liquid chromatography, the nucleic acid is isolated by precipitation from the aqueous solution. When inorganic salts are present in the purified fraction, these salts also precipitate together with the nucleic acid. Therefore, the purified fraction must be subjected to a desalting operation prior to the addition of alcohol in order to remove these salts.

The use of hydroxyapatite as the packing material of the column is a requisite in this invention. However, no special restriction on the liquid chromatographic procedure itself is required in the present invention. For example, any procedures by using conventional and simple equipment, in which the packing material is charged into a glass column and a eluent is passed through the column at lower pressure by using the difference of water head or a peristaltic pump, may be applied to this invention. Also high performance liquid chromatography using the combination of a high pressure pump and a pressure withstanding column may be applied to this invention. Any type of such equipment can suitably be used in practising this invention. High performance liquid chromatography is, in particular, preferred from the rapidness and accuracy standpoint for the separation and/or purification.

The use of aqueous solutions of aluminum salts of carbonic acid as the eluent is a requisite in this invention. The ammonium salts of carbonic acid is a volatile salt, and decomposes into ammonia and carbon dioxide under reduced pressure and/or by warming.

When the separation and/or purification of the nucleic acid is carried out by using the ammonium salt of carbonic acid, it is unnecessary to employ dialysis as used in the conventional methods. The ammonium salts of carbonic acid can be vaporized and removed in the form of ammonia and carbon dioxide within a short period of time by treating the purified fraction under a reduced pressure and/or by warming the purified fraction.

When the removal of ammonium salts of carbonic acid from the purified fraction is carried out by treating under reduced pressure, the pressure may be determined so that removal of ammonium salts of carbonic acid can be performed. The pressure which may be used is, for example, in the range of 13.3 - 133 hPa. When ammonium salts of carbonic acid are removed by warming, for example, under atmospheric pressure, the temperature is determined so as to remove the used ammonium salts of carbonic acid and to give no adverse effect on the properties of the nucleic acid to be separated. The range of temperatures for the treatment is 70°C or less and preferably 30 - 70°C, more preferably 30 - 60°C. When the treatment under reduced pressure and the warming treatment are conducted simultaneously, the treatment may be carried out, for example, under a pressure of 13.3 - 133 hPa and at a temperature of 0 to 60°C.

Among the ammonium salts of carbonic acid, ammonium hydrogen carbonate and ammonium carbamate are particularly preferred due to good solubility in alcohol. When the nucleic acid is precipitated by the addition of alcohol to the purified fraction, ammonium hydrogen carbonate or ammonium carbamate in the fraction is dissolved and remained in the alcohol solution phase and does not precipitate to contaminate the precipitate of the nucleic acid. Therefore, the nucleic acid can be effectively separated from ammonium salts of carbonic acid, even though the removal of these salts is not made or insufficiently conducted by the treatment under reduced pressure and/or the warming treatment mentioned above.

In the present invention, as mentioned above, the separation and purification of the nucleic acid is carried out by passing the aqueous solution of ammonium salts of carbonic acid through the column as the eluent. It is preferred to initiate passage of the eluent with water and to gradually raise the concentration of the ammonium salts of carbonic acid along with the progress of eluent passage. By this procedure, more sufficient separation of the nucleic acid can be made.

In the separation method of the nucleic acid according to the invention, no restriction on the molecular weight and the structure of the nucleic acid to be separated is required. Not only various nucleic acids including plasmid DNA, phage DNA, chromosomal DNA, other various DNAs, phage RNA, etc. but also nucleic acids having various molecular structures such as open-circular and closed-circular plasmids can be separated by the method of this invention. Thus the method of this invention can be suitably applied to the separation and/or purification of any type of nucleic acid.

Any pH and any concentration of the nucleic acid may be used for the solution of a mixture (sample) containing the nucleic acid to be separated by passing through hydroxyapatite. For example, the pH may be in the range of 5 - 10, preferably 6 - 9 and the concentration of the nucleic acid may be in the range of about 0.1 - 3 mg/ml.

The aqueous solution of ammonium salts of carbonic acid is used as the eluent in this invention. The ammonium salt solution has weaker eluting ability than the conventionally used aqueous solution of phosphates. However, hydroxyapatite having relatively mild adsorption force toward the nucleic acid is used as the packing material of the column. The combination of these two factors is assumed to give good results for the separation and purification of the nucleic acid.

In addition, using hydroxyapatite as the packing material enables the separation and purification of nucleic acid even dissolved in a solution containing inorganic salts such as phosphate before separation by passing through the column directly without desalting operation.

This is a great advantage of this invention and supposed to be based upon the following reasons.

(1) When hydroxyapatite is used as the packing material, the elution of the nucleic acid is not disturbed by the contaminated inorganic salts, which is different from the cases of other packing materials.

(2) Since hydroxyapatite does not retain the inorganic salts, the inorganic salts contaminating the sample are eluted without retention. Therefore, contamination by inorganic salts other than the eluent does not occur in the purified fraction.

Examples

This invention will hereinafter be described further in detail by way of examples.

Example 1

Calf thymus DNA (No. D-1501, Type-1, Product of Sigma Co., USA) was dissolved in a 10 mM potassium phosphate buffer solution in a concentration of 0.45 mg/ml. A part of the resultant solution is thermally denatured by heating and quenching. Equal amount mixture of the above resultant solution and the denatured solution thus obtained, that is, equal amount mixture of double stranded DNA and single stranded DNA was prepared and used as the sample in the following experiment.

A column having an internal diameter of 7.6 mm and a length of 100 mm was packed with hydroxyapatite. An aqueous solution of ammonium hydrogen carbonate was used as eluent. The experiment for separating and purifying nucleic acid was carried out by using high performance liquid chromatography (hereinafter abbreviated as HPLC equipment). As a result, single stranded DNA and double stranded DNA were completely separated as illustrated in Figure 1.

The equipment and operation conditions employed in the above experiment will be illustrated below in detail.

Column:   Trade Mark HCA-Column (A-7610+P-4001)

(Product of Mitsui Toatsu Chemicals Inc.)

HPLC equipment:   Model LC-6A

(Product of Shimadzu Seisakusho Ltd.)

Detection of nucleic acid:   UV 260 nm, 0.64 AUFS

Eluent:   Aqueous ammonium hydrogen carbonate solution

(pH 7.8)

| | |
|---|---|
| 0 - 0.1 min | 500 mM |
| 0.1 - 5 min | 1000 mM |
| 5 - 15 min | 1500 mM |
| | Stepwise elution |

Flow rate:   1.0 ml/min

Temperature:   Room temperature

Chart speed:   5 mm/min

Sample:   200 μl

Each fraction of single stranded DNA and double stranded DNA obtained by the operation of above chromatography was separately collected. The fractions were respectively treated at 40°C for 10 minutes under reduced pressure of 133 hPa to vaporize ammonium hydrogen carbonate. Then ethanol was added to each aqueous DNA solution thus obtained. Single stranded DNA and double stranded DNA were separately recovered as high purity precipitate.

In addition, when the ammonium hydrogen carbonate solution is used as eluent, the above operation under reduced pressure may be omitted. The present inventors have confirmed that ethanol may be directly added to each separately collected fraction to precipitate the nucleic acid which is not contaminated by the salt.

Example 2

Plasmid DNA proliferated Escherichia coli JM109 cells were collected and subjected to bacteriolysis and deproteinization treatment by an alkali-SDS method. A sample primarily containing plasmid DNA (pUC 19, Product of Takara Shuzo Co.), RNA, and chromosomal DNA was prepared and used for the experiment.

The experiment for separating the nucleic acid was carried out on the sample obtained above by the same procedures as described in Example 1.

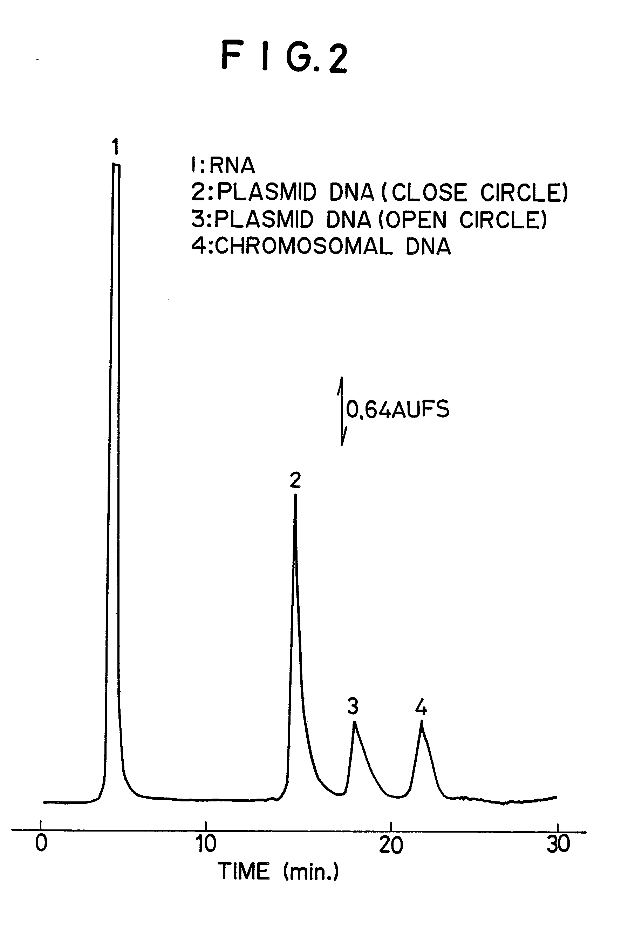

The results are illustrated in Figure 2. Plasmid DNA was completely separated from RNA and chromosomal DNA. Closed-circular Plasmid DNA and open-circular plasmid DNA were also separated, respectively.

The equipment and operation conditions employed in the above experiment will be illustrated below in detail.

```
Column:   Trade Mark, HCA-Column (A-7610+P-4001)

          (Product of Mitsui Toatsu Chemicals Inc.)

HPLC equipment:   TRI-ROTAR Model VI

                  (Product of Nihon Bunko Kogyo Co., Ltd.)

Detection of nucleic acid:   UV 260 nm, 0.64 AUFS

Eluent:   Aqueous ammonium hydrogen carbonate solution

          (pH 7.8)

          0 - 10 min          900 mM

          10 - 15 min        1000 mM

          15 - 20 min        1100 mM

          20 - 25 min        1500 mM

          Stepwise elution

Flow rate:     1.0 ml/min

Temperature:   Room temperature

Chart speed:   5 mm/min

Sample:        200 µl
```

The purified fraction of closed-circular Plasmid DNA obtained by the operation of above chromatography was collected and added with ethanol. Purified product of closed-circular Plasmid DNA was obtained as precipitate.

In addition, the purified product of above closed-circular plasmid DNA was confirmed to cause cleavage at one position by restriction enzyme (Hind III). Therefore, Plasmid DNA purified by the method of this invention can also be used for transformation.

Comparative Example 1

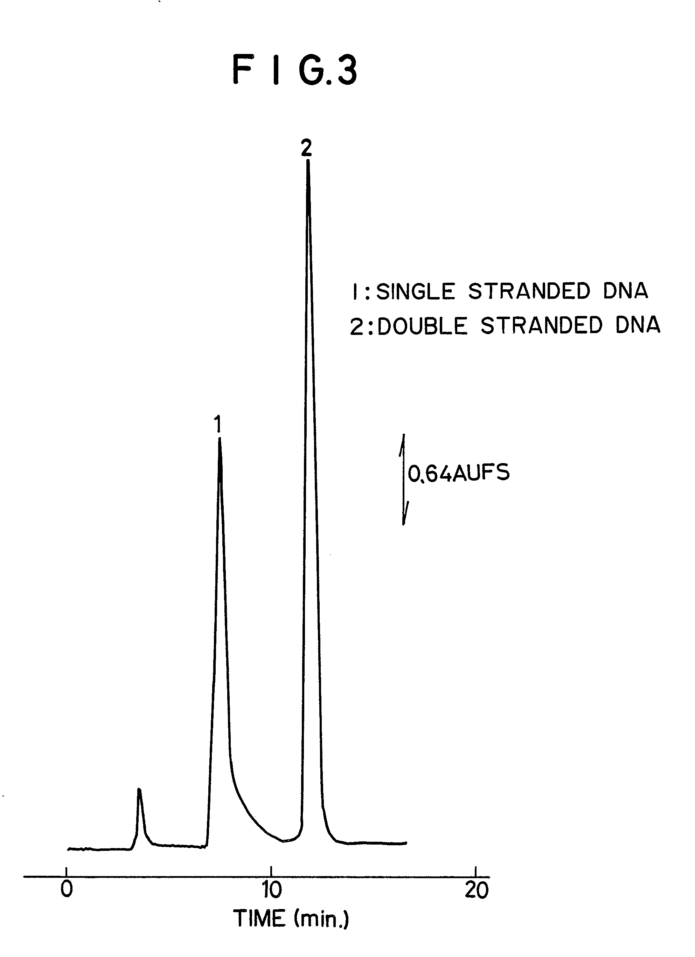

The same mixture composed of equal amount of single stranded DNA and double stranded DNA as described in Example 1 was used as the sample. The experiment for separating and purifying the nucleic acid was carried out using HPLC equipment together with combination of hydroxyapatite column and the aqueous sodium phosphate solution as eluent. The results are shown in Figure 3.

The equipment and operating conditions employed in the above experiment will be described below in detail.

Column:   Trade Mark, HCA-Column (A-7610+P-4001)

          (Product of Mitsui Toatsu Chemicals Inc.)

HPLC equipment:  Model LC-6A

                 (Product of Shimadzu Seisakusho Ltd.)

Detection of nucleic acid:   UV 260 nm, 0.64 AUFS

Eluent:   Sodium phosphate buffer (pH 6.8)

          - 0 min           100 mM

          0 - 5 min         200 mM

          5 - 15 min        300 mM

             Stepwise elution


          Flow rate:    1.0 ml/min.

          Temperature:  Room temperature

          Chart speed:  5 mm/min

          Sample:       200 μl


Each fraction of single stranded DNA and double stranded DNA obtained by the operation of above chromatography was separately collected. The fraction was respectively desalted by dialysis to remove phosphate component. DNA was precipitated and recovered by the addition of alcohol to the desalted fraction.

Long period of time was needed to complete the dialysis as follows:

Two-hour dialysis was conducted twice and then one-day dialysis was carried out twice against 0.2 M NaCl, TE solution (1 mM tris-hydrochloric acid buffer solution, 0.1 mM EDTA solution, pH 8.0).


Comparative Example 2

The same sample as prepared in Example 2 and used for the sample of liquid chromatography was used in the experiment. The separation and purification of the nucleic acid was carried out by cesium chloride equilibrium density gradient centrifugation (ultracentrifugal method). The procedures were as follows.

According to the usual method, sample, cesium chloride and ethidium bromide were poured into a centrifugal tube and subjected to an ultracentrifuge (Product of Beckman Ltd. USA) at 18°C for 40 hours at a revolution of 40,000 rpm.

After ultracentrifugation, the desired fluorescent band containing the closed-circular plasmid DNA was collected, and then the ethidium bromide was removed by three successive extraction with aqueous saturated cesium chloride solution saturated with isopropyl alcohol.

The residual portion containing the closed-circular plasmid DNA was subjected twice to two-hour dialysis. Ethanol was added to the resultant solution to recover closed-circular plasmid DNA in the form of a precipitate.

**Claims**

1. A method for separating nucleic acid comprising the steps of:
   (a) passing a solution of a mixture containing at least a nucleic acid through a column packed with granular hydroxyapatite to retain the nucleic acid in the column;
   (b) passing an aqueous solution of an ammonium salt of carbonic acid through the column retaining the nucleic acid to selectively elute the nucleic acid retained in the column; and
   (c) collecting a fraction of the eluted nucleic acid to recover the nucleic acid from the fraction.

2. The method according to claim 1, wherein a plurality of nucleic acids is contained in the mixture, retained in the column, and then they are separately eluted from the column.

3. The method according to claim 1 or claim 2, wherein the concentration of the ammonium salt of carbonic acid is in the range of 2 mM - 2 M in the solution of the ammonium salt of carbonic acid.

4. The method according to claim 3, wherein the ammonium salt of carbonic acid is ammonium carbonate, ammonium hydrogen carbonate or ammonium carbamate.

5. The method according to any of claims 1 to 4, wherein the method further comprises a step of removing the ammonium salt of carbonic acid from the eluted fraction of the nucleic acid.

6. The method according to claim 5, wherein the ammonium salt of carbonic acid is removed from the fraction by treating the eluted fraction of the nucleic acid under reduced pressure, or with warming to convert the ammonium salt to carbon dioxide and ammonia, or by warming the eluted fraction of the nucleic acid under reduced pressure.

7. The method according to claim 6, wherein the treatment under reduced pressure is in the range of 13.3 - 133 hPa (10 - 100 Torr).

8. The method according to claim 6 or claim 7, wherein the treatment with warming is at 70° C or less.

9. The method according to claim 6, wherein the treatment by warming under reduced pressure is conducted under the conditions of 13.3 - 133 hPa (10 - 100 Torr) and 0 - 60° C.

10. The method according to any of claims 1 to 5, wherein the method further comprises steps of precipitating the nucleic acid by the addition of alcohol to the eluted fraction of the nucleic acid and recovering the resultant precipitate to separate the nucleic acid from the ammonium salt of carbonic acid contained in the fraction.

11. The method according to any of claims 1 to 5, wherein the method further comprises steps of precipitating the nucleic acid by the addition of alcohol to the fraction after removing the ammonium salt of carbonic acid from the eluted fraction of the nucleic acid and recovering the resultant precipitate.

# F I G.1

1
2

I:SINGLE STRANDED DNA
2:DOUBLE STRANDED DNA

0.64AUFS

TIME (min.)

# F I G.2

1: RNA
2: PLASMID DNA (CLOSE CIRCLE)
3: PLASMID DNA (OPEN CIRCLE)
4: CHROMOSOMAL DNA

0.64 AUFS

TIME (min.)

# F I G.3

1 : SINGLE STRANDED DNA
2 : DOUBLE STRANDED DNA

0.64AUFS

TIME (min.)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | BIOCHIMICA ET BIOPHYSICA ACTA vol. 474, 1977, pages 445-455, Amsterdam, NL; H.G. MARTINSON et al.:"Thermal Elution Chromatography of Nucleic Acids on Hydroxyapatite." * complete document * --- | 1 | B 01 D 15/00 C 12 Q 1/68 |
| A | J. BIOLOGICAL CHEMISTRY vol. 234, no. 6, 1959, pages 1459-1465, Baltimore, F.M. RICHARDS et al.:"The Preparation of Subtilisin-modified Ribonuclease and the Separation of the Peptide and Protein Components." * page 1459, right hand column, lines 6-25 * ----- | 1 | |

TECHNICAL FIELDS SEARCHED (Int. Cl.4)

B 01 D 15/00
C 12 Q 1/00

The present search report has been drawn up for all claims

| Place of search | Date of completion of the search | Examiner |
|---|---|---|
| BERLIN | 31-03-1989 | BERTRAM H E H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)